# EUROPÄISCHE PATENTANMELDUNG

(11) **EP 4 445 886 A1**
(43) Veröffentlichungstag der Anmeldung: **16.10.2024**
(21) Anmeldenummer: 24000043.0
(22) Anmeldetag: 11.04.2024
(51) Int. Cl.: A61F 13/20, A61F 13/511, D04H 1/4258, D04H 1/44, D04H 1/49, D04H 1/74

(54) **ABDECKVLIES**

(30) Priorität: 13.04.2023 DE 202023000825 U
(71) Anmelder: Sandler AG, 95126 Schwarzenbach/Saale (DE)
(72) Erfinder: Bernhuber, Uwe, 95032 Hof (DE); Zeitler, Jessica, 95032 Hof (DE)
(74) Vertreter: Grünecker Patent- und Rechtsanwälte PartG mbB

(57) **Zusammenfassung**

Die Erfindung betrifft ein Abdeckvlies für Damenhygieneprodukte, das zu 100% aus Zellulose-Faserstoffen besteht, mechanisch verfestigt ist und die Oberfläche mechanisch geglättet ist.

## Beschreibung

Die Forderung nach Nachhaltigkeit in der Herstellung von persönlichen Hygieneartikeln zeigt sich in der Forderung nach Nachhaltigkeit der einzelnen Komponenten. Weiter stellt die SUPD (Single Use Plastic Directive) die Hersteller vor die Herausforderung Materialien ohne Plastikanteil einzusetzen. Auf Einwegplastikprodukte besteht aktuell eine Kennzeichnungspflicht und eine Besteuerung von Einwegprodukten mit Plastikanteil wird eingeführt werden.

Die vorliegende Erfindung betrifft Abdeckvliese für Damenhygieneartikel wie beispielsweise als Topsheetmaterialien für Slipeinlagen oder insbesondere Hüllvliesstoffe für Tampons, die für die Damenhygiene eingesetzt werden.

Stellvertretend, aber nicht beschränkend, wird anhand von Menstrualtampons der Stand der Technik erläutert.

Tampons der gattungsgemäßen Art bestehen aus
einem Abdeckvlies,
einem absorbierenden Kern
und einem Rückholfaden.

Das Abdeckvlies hat dabei mehrere Funktionen: beispielsweise muss es das leichte Einführen des Tampons gewährleisten, muss Körperflüssigkeiten zum absorbierenden

Kern hin passieren lassen und muss abstehende Fasern des absorbierenden Kerns zurückhalten.

Üblicherweise wird bei der Herstellung von Tampons ein Kardenband aus Viskose oder Mischungen aus Viskose und Baumwolle mit einem Abdeckvlies aus synthetischen Fasern im Heißsiegelverfahren verbunden. Dabei werden beispielsweise Vlieskonstruktionen mit bikomponenten Stapelfasern aus Polyethylen im Mantel und Polypropylen oder Polyethylenterephthalat im Kern eingesetzt. Während der sogenannten Versiegelung, dem Verbinden des Abdeckvlieses mit dem absorbierenden Kern, wird die niedrigschmelzende Mantelkomponente zum Verkleben genutzt. Für ein optimales Einführen des Tampons wird weiterhin ein besonders glattes Abdeckvlies bevorzugt. Solche Produkte, beschrieben in DE 19753665 C2, zeigen eine hohe Gleitfähigkeit.

Der Nachteil von thermisch verfestigten Materialien als Tamponcover ist, dass kein 100% nachhaltiger Rohstoff eingesetzt werden kann, da die Fasern über Hitze verfestigt werden. Dazu muss auf fossile Rohstoffe zurückgegriffen werden. Ein weiterer Nachteil dieser Materialien ist der steife, synthetische Griff.

In EP 3943054 B1 und in EP 2776619 B1 sind Vliesstoffe beschrieben, die zu 100% aus biologisch abbaubaren, nachhaltigen Fasern bestehen. Diese Vliesstoffe können als Abdeckvlies für einen Menstrualtampon verwendet werden. Hier wurde bereits erkannt, dass ein Abdeckvlies aus 100% nachhaltigen Fasern keine gute Gleitfähigkeit aufweist. Die EP 22776619 B1 sieht daher eine chemische Behandlung der zur Anwendung kommenden Faserstoffe zur Verbesserung der Oberflächenglätte vor. Eine zusätzliche chemische Behandlung von Vliesstoffen als Hülle eines Tamponkerns ist vom Endverwender nicht gewünscht.

Die Aufgabe der vorliegenden Erfindung ist es daher, ein Abdeckvlies aus zellulosischen Faserstoffen für Damenhygiene-Produkte bereitzustellen, welches aus 100% nachhaltigen Faserstoffen besteht und eine glatte und ebene Oberflächenstruktur besitzt.

Die Lösung der Aufgabe geschieht anhand der Merkmale der Ansprüche 1-6

Ein erfindungsgemäß hergestelltes Abdeckvlies wird aus 100% zellulosischen Fasern mittels Kardiertechnik hergestellt und mechanisch mittels Nadelung, bevorzugt durch das Spunlace-Verfahren verfestigt. Unter dem Begriff Spunlace wird die Wasserstrahlverfestigung verstanden.

Ein rein zellulosisches Spunlace-Vlies eignet sich aufgrund seiner Dicke und seiner unebenen Struktur nur bedingt für ein Tamponcover. Um die Funktionsweise eines Spunlace Materials entsprechend zu beeinflussen, erfolgt eine nachträgliche Kalandrierung des Material.

Mittels der Kalandrierung wird das Spunlace-Vlies geglättet und dessen Dicke verringert. Ein so behandeltes Vlies kann dann als Abdeckvlies, bspw als Hüllvlies für einen Tampon oder als Topsheet für Slipeinlagen oder ähnliches eingesetzt werden.

Somit können 100% nachhaltige Fasermaterial zur Herstellung genutzt und trotzdem die Eigenschaften eines thermisch verfestigten Materials nachgestellt werden.

Zur Untersuchung kamen folgende Prüfmethoden zur Anwendung:
Flächengewicht gemäß WSP 130.1., Angabe in g/m²
Dicke gemäß WSP 120.6, Messdruck 0,5kPa, Angabe in mm
(Die WSP-Methoden sind bei der EDANA, B-1160 Brussels, Belgien erhältlich)

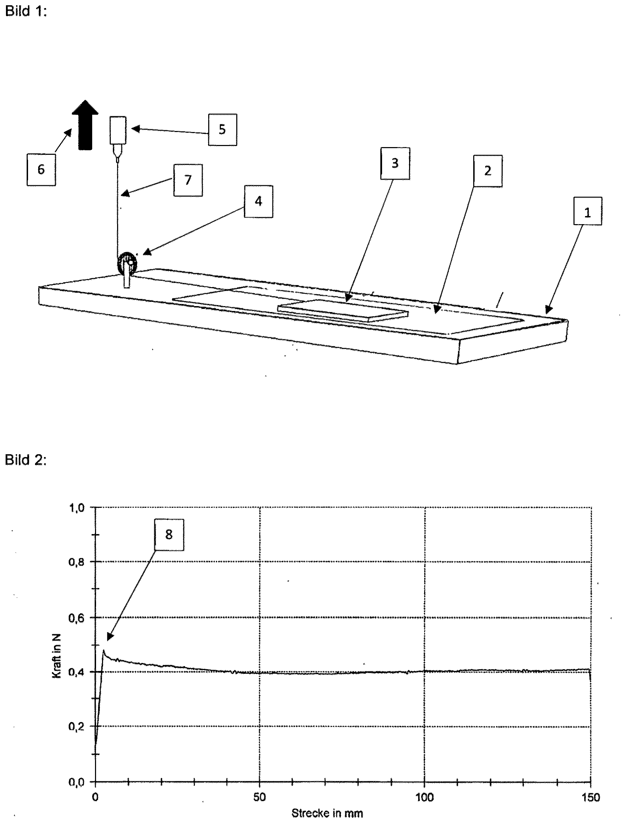

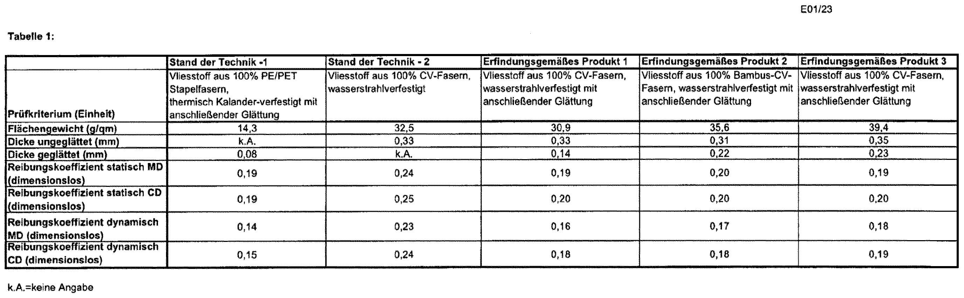

Reibungskoeffizient gemäß DIN EN ISO8295 -10/2004, Angabe als dimensionsloser Quotient aus Reibungskraft (in N) und vom Testschlitten (3) ausgeübte Normalkraft (1,96N). Der Testaufbau kann dem Bild 1 entnommen werden. Im Rahmen der vorliegenden Erfindung wurde der statische und der dynamische Reibungskoeffizient wie folgt ermittelt.

| | |
|---|---|
| Maße des Schlittens (3): | quadratische Metallscheibe mit Kantenlänge 63,5mm * 63,5mm |
| Gewicht des Schlittens (3): | 200g, entsprechend einer Normalkraft von 1,96N |
| Oberfläche des Schlittens (3): | Stahl, poliert |
| Testgeschwindigkeit: | 127mm/min |
| Testlänge: | 150mm |
| Mustergröße: | 150 * 300mm |

Abweichend von der DIN EN ISO 8295 wird der Schlitten (3) in Bewegung gebracht, der Prüftisch (1) nicht. Dazu wird das Muster (2) auf dem Prüftisch (1) faltenfrei fixiert, der Schlitten (3) am Anfangspunkt der Prüfung aufgesetzt. Der Schlitten (3) ist mittels einer dehnungsfreien Polyamid-Schnur (7) über die reibungsarme Umlenkrolle (4) mit der Kraftmessdose (5) verbunden. Der Schlitten (3) wird mittels Bewegung der Kraftmessdose (6) in Bewegungsrichtung (6) gezogen. Dabei wird der Schlitten (3) über die gesamte Testlänge von 150mm mit konstanter Testgeschwindigkeit von 127mm/min gezogen. Die jeweils anliegende Kraft wird mit der Kraftmessdose (5) aufgezeichnet.

Das Bild 2 zeigt einen beispielhaften Verlauf der Kraftkurve über die Testlänge. Die Y-Achse zeigt die an der Kraftmessdose (5) anliegende Kraft in N, die X-Achse die Testlänge in mm.

Die innerhalb der ersten 10mm Testlänge beobachtete Maximalkraft (8) wird als statische Reibungskraft Fs aufgenommen. Die mittlere Kraft, die sich für die Bewegungen des Schlittens auf der Teststrecke nach den ersten 10mm bis zum Ende der Teststrecke bei 150mm ergibt, wird als dynamische Reibungskraft Fd bezeichnet.

Die jeweiligen Reibungskoeffizienten werden dann gemäß der EIN EN ISO 8295:2004 gemäß den Formeln aus den Abschnitten 3.4.1. (statischer Reibungskoeffizient) und 3.4.2 (dynamischer Reibungskoeffizient) errechnet.

Nachhaltige Rohmaterialien oder nachhaltige Faserstoffe definieren sich im Rahmen dieser Erfindung als zellulosische Fasern aus regenerativ gewonnener Zellulose wie beispielsweise Viskosefasern. Sie bestehen aus Polymerketten des Makromoleküls Zellulose, wobei die Polymerketten üblicherweise einen durchschnittlichen Polymerisationsgrad von 200-600 aufweisen. Handelsübliche Viskosefasern für die Textilindustrie haben üblicherweise auch hydrophile Eigenschaften. Die Sinkzeit beträgt üblicherweise <1 Minuten. Zur Herstellung der erfindungsgemäßen Abdeckvliese werden zellulosische Fasern mit einer Feinheit zwischen 1,3 - 2,9 dtex in Ballenform verwendet. Diese zellulosischen Fasern sind kommerziell beispielsweise bei den Firmen Lenzing AG oder Kelheim erhältlich.

Die nachstehenden Erläuterungen beziehen sich stellvertretend auf wasserstrahlverfestigte Vliesstoffe, andere mechanische Verfestigungsmethoden sind vorstellbar.

Trockenverfahren: Die Herstellung des Grundvliesstoffes für erfindungsgemäße Abdeckvliese können nach einem im Buch "Vliesstoffe", erschienen im Verlag Wiley VCH, 2. Auflage, 2012 aufgezeigten Trockenverfahren hergestellt werden. Erfindungsgemäß bevorzugt, aber ohne darauf beschränkt zu sein, wird das Kardierverfahren unter Verwendung von Stapelfasern.

Wasserstrahlverfestigung: bezeichnet ein Verfestigungsverfahren für erfindungsgemäße Deckblätter. Die grundlegenden Techniken dazu werden im Buch "Vliesstoffe", erschienen im Verlag Wiley VCH, 2. Auflage, 2012, Seiten 340-359 beschrieben.

Kalandrierung: für die Kalandrierung wird ein üblicher Textilkalander aus der Textilveredlung eingesetzt. Der Kalander besteht üblicherweise aus zwei drehenden Walzen, die mit Druck aufeinander gepresst und nicht geheizt werden.

Ein erfindungsgemäßes Abdeckvlies besteht zu 100% aus Zellulose-Fasern. Ein derartiges Abdeckvlies kann, ohne darauf festgelegt zu sein, wie folgt hergestellt werden:
- Öffnen und Mischen der Faserballen zellulosischer Fasern
- Bildung eines unverfestigten Faserflores, bspw mittels Kardieren
- Verfestigen des Faserflors mittels mechanischer Verfestigung durch beispielsweise Nadeln oder Wasserstrahlen
- Trocknen des Vliesstoffes
- Glätten der Oberfläche des Vliesstoffes / Verdichten durch Kalanderpassage

Die überschüssige Feuchtigkeit, die mittels der Verfestigungsart Wasserstrahl, durch die Verfestigung in das Vlies eingetragen wird, wird mit einem nachfolgenden Trocknungsverfahren entfernt, sodass der so erfindungsgemäß hergestellt Vliesstoff eine Restfeuchte von 7 bis 13 Gewichts % aufweist.

Erfindungswesentlich ist eine nachfolgende Kalanderpassage des zuvor gebildeten Vliesstoffes. Dabei wird das Vlies durch den Spalt eines Kalanders, bspw eines Zwei-Walzenkalanders mit Stahl/Stahl oder Stahl/Gummi-Walzen geführt. Die Walzen drücken dabei mit einem Liniendruck von 125daN/cm aufeinander.

Das Flächengewicht eines so hergestellten Vliesstoffes liegt je nach Konstruktion des Hygieneartikels im Bereich von 30 bis 50 g/m², bevorzugt bei 30 bis 40g/m².

Aus der Tabelle 1 können folgende Daten abgelesen werden:
Ein Vlies-1 nach dem Stand der Technik der DE 19753665 C2, gebildet aus 100% bikomponenten Faser mit Polyester Kern und Polyethylen Hülle, wurde nach der Faserflorbildung vollflächig mit Druck und Hitze verfestigt. Das Vlies weist einen papierartigen Griff auf, der statische Reibungskoeffizient liegt bei 0,19 in beiden Ausrichtungen in Querrichtung (CD) und Längsrichtung (MD). Der dynamische Reibungskoeffizient liegt in MD bei 0,14, in CD bei 0,15. Die ermittelten Reibungskoeffizienten stellen den allgemein üblichen Standard von Abdeckvliesen als Hüllvliesen für die Verwendung im Bereich Menstrualtampon dar. Aufgrund der eingesetzten Faserstoffe ist ein derartiges Vlies nicht biologisch abbaubar und der Fokus auf nachhaltige Produkte kann nicht gewährleistet werden.

Ein Vlies -2 besteht aus 100% Viskosefasern. Im Vergleich zu Vlies 1 hat das Vlies-2 eine höhere Dicke und auch deutlich höhere Reibungskoeffizienten, sowohl statisch als auch dynamisch. Das Vlies ist zwar biologisch abbaubar, aufgrund der Dicke und auch der hohen Reibungskoeffizienten ist das Vlies-2 aber für den Herstellprozess eines Menstrualtampons nicht geeignet. Auch bei der Herstellung von Slipeinlagen führen die hohen Reibkoeffizienten zu Problemen bei der Verpackung, da die einzelnen Slipeinlagen nicht faltenfrei in die Verpackung gleiten.

Bei den erfindungsgemäß ausgeführten Vliesstoffen 3, 4 und 5 werden jeweils biologisch abbaubare Faserstoffe eingesetzt. Vlies 3 und 5 bestehen aus Viskose-Fasern, deren Zellulose aus Bäumen gewonnen wurde, Vlies 4 besteht aus Viskose-Fasern, deren Zellulose aus Bambus gewonnen wurde.

Bei den erfindungsgemäß ausgeführten Vliesstoffen 3, 4 und 5 wurde jeweils eine Kalanderbehandlung durchgeführt. Die Kalanderbehandlung erfolgte mittels eines Zwei-Walzenkalanders Stahl/Gummi-Walzen mit Liniendruck von 125daN/cm und einer Geschwindigkeit von 25 m/min. beaufschlagt.

Das erfindungsgemäß ausgeführte Vlies 3 hat im Vergleich zu Vlies 2 nach der Kalandrierung eine um 58% geringere Dicke, der statische Reibungskoeffizient liegt 20% geringer, der dynamische Reibungskoeffizient 30% geringer. Das erfindungsgemäß wasserstrahlverfestige und kalandrierte Vlies 3 liegt damit im Bereich der Reibkoeffizienten von Vlies 1.

Die erfindungsgemäß ausgeführten Vliese 4 und 5 haben höhere Vliesgewichte und wurden mit der gleichen Kalanderbehandlung, durch zwei Walzen mit einem Liniendruck von 125daN/cm bei einer Geschwindigkeit von 25 m/min beaufschlagt. Überraschend zeigte sich, dass nicht die Geschwindigkeit sondern der Druck ausschlaggebend für die Erzielung der Oberflächenglätte ist.

Auch hier zeigt sich eine deutliche Verringerung der Reibungskoeffizienten, sodass der statische Reibungskoeffizient unter 0,20 und der dynamische Reibungskoeffizient unter 0,18 liegt.

So erfindungsgemäße ausgeführte Vliesstoffe sind als Abdeckvliese für Menstrualtampons aber auch als Topsheet für Slipeinlagen, Damenbinden oder ähnliches geeignet. Sie vereinen die Forderung nach Gleitfähigkeit, ausgedrückt als Reibungskoeffizienten kleiner 0,20 mit der
- Nutzung von 100% nachhaltigen Rohstoffen
- Reduzierung der unebenen Oberflächenstruktur und Verbesserung der Weichheit des Materials
- Verbesserung des Fluidmanagements hinsichtlich des Einzugs von Fluiden
- Verbesserung der Herstellung, insbesondere bei Verpackung von Slipeinlagen
- Wegfall des Schildkrötensymbols (SUPD) auf der Verpackung

### Bezugszeichenliste:

- 1 -: Prüftisch
- 2 -: Muster
- 3 -: Schlitten
- 4 -: Umlenkrolle
- 5 -: Kraftmessdose
- 6 -: Bewegungsrichtung
- 7 -: Polyamid-Schnur
- 8 -: Maximalkraft

## Patentansprüche

1. Abdeckvlies für Damenhygieneprodukte, bestehend aus einem mechanisch verfestigten Stapelfaservlies, das zu 100% aus Zellulose-Faserstoffen besteht
**dadurch gekennzeichnet, dass**
die Oberfläche des Abdeckvlieses mechanisch geglättet ist,
das Abdeckvlies einen statischen Reibungskoeffizient von kleiner 0,20 aufweist und
das Abdeckvlies einen dynamischen Reibungskoeffizient von kleiner 0,18 aufweist.

2. Abdeckvlies nach Anspruch 1,
**dadurch gekennzeichnet, dass**
dass die Zellulose-Fasern Viskose-oder Lyocell- Fasern sind.

3. Abdeckvlies nach den vorstehenden Ansprüchen,,
**dadurch gekennzeichnet, dass**
die Oberfläche des Hüllvlieses mittels glatten Kalanderwalzen aus Stahl und/oder Gummi geglättet ist.

4. Abdeckvlies nach den vorstehenden Ansprüchen,
**dadurch gekennzeichnet, dass**
das Abdeckvlies mittels Wasserstrahlvernadelung verfestigt wurde.

5. Tampon für Hygienezwecke bestehend aus einem Abdeckvlies, einem Saugkern und einem Rückholfaden,
**dadurch gekennzeichnet, dass**
das Abdeckvlies gemäß der Ansprüche 1 - 4 gebildet wird.

6. Slipeinlage oder Damenbinde beinhaltend ein Abdeckvlies
**dadurch gekennzeichnet, dass**
das Abdeckvlies gemäß der Ansprüche 1 - 4 gebildet wird.
